Europäisches Patentamt

(19) European Patent Office

Office européen des brevets

(11) Publication number: **0 228 353 B1**

# EUROPEAN PATENT SPECIFICATION

(45) Date of publication of patent specification: **15.05.91**

(51) Int. Cl.⁵: **A61F 5/443, A61F 5/453, A61F 5/455, A61F 13/00, A61L 15/00**

(21) Application number: **86850196.6**

(22) Date of filing: **02.06.86**

(54) **A device for collecting and absorbing liquid, and a method of manufacturing an absorption and spreading layer intended for liquid.**

(30) Priority: **20.12.85 US 811676**

(43) Date of publication of application:
**08.07.87 Bulletin 87/28**

(45) Publication of the grant of the patent:
**15.05.91 Bulletin 91/20**

(84) Designated Contracting States:
**AT BE CH DE FR GB IT LI LU NL SE**

(56) References cited:
**EP-A- 0 108 637**
**DE-A- 3 137 839**
**GB-A- 2 132 897**
**US-A- 4 338 371**
**US-A- 4 435 178**

**A BILAGA TILL SVENSK PATENT TIDNING (supplement to Swedish Patent Gazette) No. 6, February 11, 1985, Stockholm, Sweden page 3**

(73) Proprietor: **Mattsson, Lars**
**Utäng 601**
**S-440 60 Skärhamn(SE)**

(72) Inventor: **Mattsson, Lars**
**Utäng 601**
**S-440 60 Skärhamn(SE)**

(74) Representative: **Barnieske, Hans Wolfgang c/o H.W. Barnieske Patentbyrä AB P.O. Box 25 Turingegatan 26 S-151 21 Södertälje 1(SE)**

## Description

The present invention relates to a device for collecting and absorbing liquid, particularly urine, comprising at least one absorption body of super-absorbent type which, upon absorption of liquid forms an expandable gel, the device comprising a laminate composed of several layers in which there is a first layer of substantially liquid-proof material in combination with a second layer of material permeable to liquid, which form at least one space enclosing a first absorption body, a layer having an open-celled structure arranged in a first said space to spread the liquid over a first said absorption body arranged in said first space, to avoid gel blocking the layer of open-celled structure consisting of a substantially elastic non-absorbent material wherein the liquid-proof material consists of a plastic layer constituting one side of the multi-layer laminate and wherein the material permeable to liquid consists of a non-woven fabric constituting the other side of the laminate, the absorption body and the elastic material which consists of a foam plastic being enclosed in the space therebetween, a second absorption body which is provided between the non-woven fabric and the first absorption body, and an inner, substantially liquid-proof third layer which is arranged at least partially between the layer of open-celled structure and the liquid-permeable layer, to form a second space holding said second absorption body.

The object of the present invention is to eliminate the drawbacks of the known device of collecting and absorbing urine and achieve a product of the type described in the introduction, which will provide reliable absorption of urine without risk of over-filling, while at the same time minimizing the dimensions of the product.

This is achieved with the device according to the invention which is defined in claim 1. Embodiments of the invention are defined in claim 2 and 3.

The device may be substantially triangular in shape and the multilayer laminate may be joinined subsequent to shaping to give the device a premanent, preferably concave, upwardly curved form.

The outer liquid-proof layer, the first absorption body and the layer of open-celled or porous structure may be integrated to a layer. The super-absorbent polymer powder or granular material may be incorporated in said integrated layer, which may be manufactured by a method in which a layer having a open-celled or porous structure is caused to adhere to a substantially liquid-proof material, perferably a plastic foil, after which a super-absorbent polymer powder or granular material is added and caused to penetrate into the layer.

The super-absorbent polymer powder or granular material is suitably caused to adhere to the cells of the layer and to the plastic foil due to the addition of a binder, the layer being passed through a mist of binder prior to supplying the super-absorbent polymer powder or granular material.

Penetration of the super-absorbent polymer powder or granular material can be achieved by shaking or vibrating the layer and said layer may consist of foam plastic having open cells, such as foamed polythene.

The liquid-proof material may be provided with a coating of binder for adhesion to the layer having cellular or porous structure, or the said layer may be formed by means of foaming directly on the liquid-proof material, thus adhering thereto.

The invention will be further described in the following with reference to the accompanying drawing which shows a sectional view, seen from the side, through a device according to the invention as used by a woman.

The embodiment shown in the drawing a device 1 for collecting and absorbing liquid, preferably urine, comprises a laminate composed of several layers in which a substantially liquid-proof material 2 is combined with a liquid-permeable material 3 to form at least one space 4, 5, enclosing an absorption body 6, 7. The device 1 also includes a layer 8, having opencelled structure.

The device 1 shown thus has an outer, plastic layer 9, a first absorption body 6, a layer 8 of opencelled structure, an inner plastic layer 10, a second absorption body 7 and an outer liquid-permeable material 3, an opening or recess 11 being provided through the inner plastic layer 10 and the second absorption body 7. The plastic layers 9 and 10 are joined together with the outer, liquid-permeable material 3 along an outer defining edge 12, for example by welding or gluing.

The liquid-permeable materials 3 suitably consist of a thin fibre material, as non-woven or woven fabric, through which the urine can pass. The substantially liquid-proof material 2 consists of the plastic layers 9 and 10 of a polythene foil or similar plastic material, which are substantially liquid-proof. The layer 8 having a cellular or porous structure suitably consists of a substantially elastic, non-absorbent material which is somewhat thicker than the other layers, such as a foam plastic having open cells with a thickness of 3 - 5 mm. The absorption bodies 6 and 7 are preferably of super-absorbent type, such as a laminate consisting of soft paper or tissue integrated primarily with a super-absorbent polymer which forms an expandable gel upon absorption of liquid. Different material combinations are also possible for the absorption body 6, such as non-woven fabric, fluff or combinations of these.

According to an alternative embodiment of the outer plastic layer 9, the first absorption body 6 and the layer 8 having a cellular or porous structure may be replaced by a single integrated layer. This layer may consist of foamed polythene which is foamed directly onto a plastic film coated with binder. The foamed polythene is then coated with a binder in mist form, whereupon super-absorbent polymer powder or granular material is added and adheres to the binder. Alternatively a thin non-woven fabric could be used to cover the polymer powder and hold it to be foamed polythene.

Upon evacuation, therefore, the urine passes through the liquid-permeable material 3 and is spread via the layer 8 to the absorption body 6. The super-absorbent laminate will then mix with the urine to form an expandable gel. Upon continued evacuation, the urine will be spread mechanically by the layer over the whole absorption body 6.

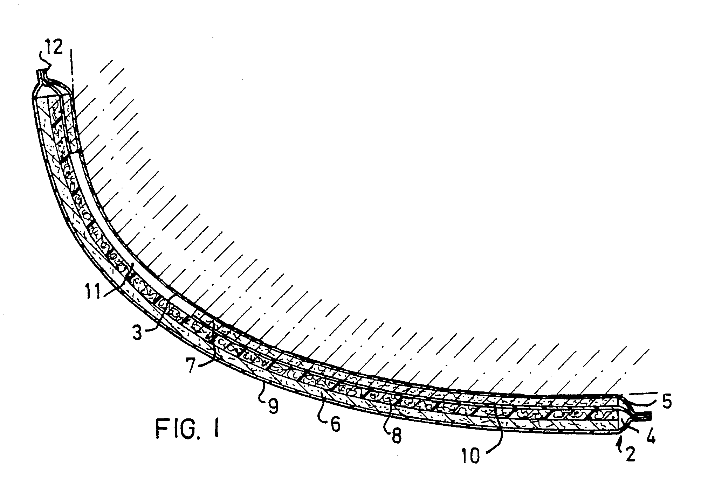

The device according to Figure 1 is intended for use by women. It is substantially triangular and is intended to be applied directly against the urine-evacuating organ with one angle directed downwardly.

The device 1 permits a volume increase in its thickness. The opening or recess 11 through the plastic layer 10 and absorption body 7 is suitably oval in shape so that the device can be made available in a single size designed to fit different individuals. The device 1 can be secured by means of double-sided tape for application on tightfitting briefs or the like. The device 1 may be given a permanent concave, upwardly curved form at the manufacturing stage. This will give a better fit and, when used with tight-fitting briefs, the elastic layer 8 will be compressed, thus providing substantially sealing contact against the woman's urine-evacuating organ. In order to further reduce the risk of leakage, the device 1 is provided with a second absorption body 7, also of superabsorbent material, so that in the event of leakage this absorption body 7 will swell and fill out any unevenness, folds, etc. This effect is achieved over the entire absorption surface since the liquid-permeable material 3 does not impede passage of the urine.

Different material combinations are also possible for the absorption body 6, such as tissue, fluff or superabsorbent polymer powder or granular filler, or combinations of these.

In order to achieve a thinner product the layer having open-celled structure can be adhered to a plastic foil, as described earlier, after which a super-absorbent polymer powder or granular material is added and caused to penetrate into the layer of cellular or porous structure. The layer having open-celled structure may consist of foam polythene which has been foamed directly on a plastic film, or has been adhered to a plastic film. Penetration of the super-absorbent polymer powder or granular material can be achieved by shaking or vibrating the layer in suitable manner. The layer having open-celled structure may also be provided with a coating of binder so that the powder or granular material will adhere substantially filling the open cells. Of course, the powder or granular material can be held in position by covering the open-celled structure with non-woven fabric or the like.

The second absorption body should preferably have a high liquid absorption capacity, but should not spread the liquid unevenly, since the first absorption body should have a large spreading of the liquid. The first absorption body should at least absorb 75% of the liquid and the second absorption body should absorb up to 25% of the liquid. The second absorption body consists of a thin supporting material having a large quantity of superabsorbent polymer powder. Upon evacuation, therefore, the urine flowing outside of the opening or recess, will be absorbed by the second absorption body, the expanding gel of which, will result in a gel-blocking around the opening or recess. If the second absorption body is provided with a sufficient amount of super-absorbent polymer powder, the gel-blocking will be so effective, that the second absorption body will provide a selfsealing action, eventhough the liquid penetrate underneath. The invention is of course not limited to the embodiments shown in the drawing but can be varied in many ways within the scope of the following claims.

## Claims

1.  A device for collecting and absorbing liquid, particularly urine, comprising at least one absorption body (6, 7) of super-absorbent type which, upon absorption of liquid forms an expandable gel, the device (1) comprising a laminate composed of several layers, in which there is a first layer of substantially liquid-proof material (2) in combination with a second layer of material permeable to liquid (3), which form at least one space (4, 5) enclosing a first absorption body (6), a layer (8) having an open-celled structure arranged in a first said space (4) to spread the liquid over a first said absorption body (6) arranged in said first space (4) to avoid gel blocking, the layer (8) of open-celled structure consisting of a substantially elastic non-absorbent material wherein the liquid-proof material (2) consists of a plastic layer (9) constituting one side of the multi-layer laminate and wherein the material (3) permeable to liquid consists of a non-wove fabric constituting the other side of the laminate, the first said absorption body (6) and the elastic material (8), which consists of a foam plastic being enclosed in the space (4) therebetween, a second absorption body (7) which is provided between the non-woven fabric (3) and the first said absorption body (6), and an inner, substantially liquid-proof third layer (10) which is arranged at least partially between the layer (8) of open-celled structure and the liquid-permeable layer (3), to form a second space (5) holding said second said absorption body (7), **characterized** in that an opening or recess (11) extends through the second said absorption body (7) and the inner, liquid-proof layer (10).

2. A device according to claim 1, **wherein** said device (1) is substantially triangular in shape and wherein the multi-layer laminate is joined subsequent to shaping to give the device a permanent, preferably concave, upwardly curved form.

3. A device according to claim 1 or 2, **wherein** the outer, liquid-proof layer (9), the first absorption body (6) and the layer (8) of opencelled or porous structure are integrated into a layer.

**Revendications**

1. Dispositif destiné à collecter et à absorber un liquide, notamment de l'urine, comprenant au moins un corps d'absorption (6, 7) du type superabsorbant, lequel, au moment de l'absorption du liquide forme un gel expansible, le dispositif (1) comprenant une stratification constituée de plusieurs couches, dans laquelle la première couche est essentiellement en un matériau étanche au liquide (2) en combinaison avec une seconde couche en un matériau perméable au liquide (3), qui forment au moins un espace (4, 5) renfermant un premier corps d'absorption (6), une couche (8) ayant une structure à alvéoles ouverts disposée dans un premier espace (4) de façon à étaler le liquide sur un premier corps d'absorption (6) disposé dans le premier espace (4) pour éviter le blocage du gel, la couche (8) de structure à alvéoles ouverts consistant en un matériau non-absorbant sensiblement élastique, dans lequel le matériau étanche au liquide (2) consiste en une couche plastique (9) constituant un côté de la stratification multicouche et dans lequel le matériau (3) perméable au liquide consiste en une nappe de non-tissé constituant l'autre côté de la stratification, le premier corps d'absorption (6) et le matériau élastique (8) qui consiste en un plastique expansé ou alvéolaire étant renfermé dans l'espace (4) intermédiaire, un second corps d'absorption (7) qui est disposé entre la nappe de non-tissé (3) et le premier corps d'absorption (6), et une troisième couche interne, sensiblement étanche au liquide (10), qui est disposée au moins partiellement entre la couche (8) de structure à alvéoles ouverts et la couche perméable au liquide (3) pour former un second espace (5) maintenant en place le second corps d'absorption (7), caractérisé en ce qu'une ouverture ou évidement (11) s'étend à travers le second corps d'absorption (7) et la couche intérieure, étanche au liquide (10).

2. Dispositif selon la revendication 1, dans lequel le dispositif (1) est de forme sensiblement triangulaire et dans lequel la stratification multicouche est assemblée après un formage pour conférer au dispositif une configuration permanente courbée vers le haut, de préférence concave, permanent.

3. Dispositif selon la revendication 1 ou 2, dans lequel la couche extérieure étanche au liquide (9), le premier corps d'absorption (6) et la couche (8) de structure poreuse ou à alvéoles ouverts sont intégrés dans une couche.

**Ansprüche**

1. Gerät zum Sammeln und Absorbieren von Flüssigkeit, insbesondere Urin, enthaltend mindestens einen Absorptionskörper (6, 7) aus superabsorbierendem Werkstoff, der bei Absorption der Flüssigkeit ein schäumbares Gel bildet, das Gerät (1) enthält ein aus mehreren Schichten zusammengesetztes Laminat, in dem eine erste Schicht aus im wesentlichen flüssigkeitsundurchlässigem Werkstoff (2) in Kombination mit einer zweiten Schicht aus flüssigkeitsdurchlässigem Werkstoff (3) mindestens einen Zwischenraum (4, 5) bildet, der einen ersten Absorptionskörper (6) umschließt, eine in einem ersten der besagten Zwischenräume (4) angeordnete Schicht (8) mit offenzelliger Struktur zur Verteilung der Flüssigkeit über einen ersten der besagten in besagtem ersten Zwischenraum (4) angeordneten Absorptionskörper (6) zur Vermeidung der Gel-Blockierung, wobei die offenzellig strukturierte Schicht (8) aus einem im wesentlichen elastischen, nichtabsorbierenden Werkstoff besteht, worin das flüssigkeitsundurchlässige Material (2) aus einer Kunststoffschicht (9) besteht, die eine Seite des mehrschichtigen Laminats bildet, und worin das flüssigkeitsdurchlässige Material (3) aus einem Vliesstoff besteht, der die andere Seite des Laminats bildet, den ersten der besagten Absorptionskörper (6) und das aus einem Schaumkunststoff bestehende elastische Material (8), das von dem Zwischenraum (4) dazwischen umschlossen wird, einen zweiten Absorptionskörper (7) zwischen dem Vliesstoff (3) und dem ersten der besagten Absorptionskörper (6), und einer im wesentlichen flüssigkeitsundurchlässigen dritten Schicht (10), die mindestens teilweise zwischen der offenzellig strukturierten Schicht (8) und der flüssigkeitsdurchlässigen Schicht (3) zur Formung eines zweiten Zwischenraums (5) für die Aufnahme des zweiten der besagten Absorptionskörper (7) angeordnet ist, dadurch gekennzeichnet, daß sich eine Öffnung oder ein Einstich (11) durch den zweiten der besagten Absorptionskörper (7) und die flüssigkeitsundurchlässige Schicht (10) erstreckt.

2. Gerät nach Anspruch 1, wobei besagtes Gerät (1) in der Form im wesentlichen dreieckig ist, und in welches das mehrschichtige Laminat nach der Formgebung eingesetzt wird, damit das Gerät eine stetige, vorzugsweise konkave, nach oben gekrümmte Form erhält.

3. Gerät nach Anspruch 1 oder 2, worin die flüssigkeitsundurchlässige Außenschicht (9), der erste Absorptionskörper (6) und die offenzellig strukturierte oder poröse Schicht (8) in eine Schicht integriert sind.

FIG. 1